# EUROPEAN PATENT APPLICATION

(11) **EP 4 640 202 A1**
(43) Date of publication of application: **29.10.2025**
(21) Application number: 24184589.0
(22) Date of filing: 26.06.2024
(51) Int. Cl.: A61K 8/25, A61K 8/26, A61Q 1/02

(54) **MILITARY CAMOUFLAGE CREAM AND MILITARY CAMOUFLAGE CREAM PACKAGE INCLUDING THE SAME**

(30) Priority: 25.04.2024 KR 20240055436
(71) Applicant: Defense Agency for Technology and Quality, Jinju-si, Gyeongsangnam-do 52851 (KR)
(72) Inventor: Kim, Young Hwa, 08610 Seoul (KR); Park, Yun Sun, 06289 Seoul (KR); Lee, Dong Hun, 04139 Seoul (KR)
(74) Representative: Petraz, Gilberto Luigi

(57) **Abstract**

The present disclosure relates to a military camouflage cream and a military camouflage cream package including the same, and more specifically, to a military camouflage cream containing silica, talc and kaolin, the contents of which may be controlled to exhibit adhesion, skin regeneration, skin cleansing ability, an antibacterial effect, pore contraction, and dead skin cell removal, as well as a matte effect without being greasy due to excellent sweat absorption, and a military camouflage cream package including the same.

## Description

### BACKGROUND

### 1. Technical Field

The present disclosure relates to a military camouflage cream and a military camouflage cream package including the same, and more specifically, to a military camouflage cream containing silica, talc and kaolin, the contents of which may be controlled to exhibit adhesion, skin regeneration, skin cleansing ability, an antibacterial effect, pore contraction, and dead skin cell removal, as well as a matte effect without being greasy due to excellent sweat absorption, and a military camouflage cream package including the same.

### 2. Related Art

Soldiers apply camouflage cream having a color similar to the surrounding environment to their faces to prevent exposure to the enemy during military action or guard duty. This camouflage cream is made by mixing pigments and lipophilic substances such as wax.

Existing camouflage creams not only cause various side effects by accelerating the phenomenon of chapped skin while pulling the skin severely in the winter, and blocking the discharge of sweat by blocking skin pores in the summer, but also have a problem in that they are not easily removed by soap when washing the face.

As such, camouflage creams that are used by soldiers do not take into account the skin at all and focus on camouflage so as not to be exposed to the enemy as much as possible. Thus, today, female soldiers or new generation soldiers are avoiding the use of these creams.

Camouflage creams to be used for concealment or tactical purposes should have a certain color value and excellent camouflage performance and should be formulas that are hypoallergenic to the skin.

Existing camouflage creams have problems in that the camouflage performance thereof is reduced due to their gloss that is greater than the surrounding area, and in that they are not easily washed out in the process of removing the same after camouflage, causing skin problems.

Therefore, studies are needed to establish color values suitable for tactical terrain, season, and climate, basic raw materials causing no skin trouble, and the pigment mixing ratio for camouflage function, thereby providing a camouflage cream that exhibits natural camouflage performance, has smooth spreadability by containing kaolin, is washed off without pigmentation on the skin when washing, and causes less skin problems.

### SUMMARY

An object of the present disclosure is to provide a military camouflage cream that contains silica, talc, and kaolin, the contents of which may be controlled to improve spreadability, opacity, camouflage performance, and washing-out ability, and prevent skin problems, and a military camouflage cream package including the same.

However, objects to be achieved by the present disclosure are not limited to the above-mentioned problems, and other problems not mentioned above will be clearly understood by those skilled in the art from the following description.

One embodiment of the present disclosure provides a military camouflage cream containing silica, talc, and kaolin.

According to one embodiment of the present disclosure, the content of kaolin may be 1.6 parts by weight to 2.8 parts by weight based on 100 parts by weight of the military camouflage cream.

According to one embodiment of the present disclosure, the content of silica may be 5.0 parts by weight to 9.0 parts by weight based on 100 parts by weight of the military camouflage cream, and the content of talc may be 3.5 parts by weight to 7.5 parts by weight based on 100 parts by weight of the military camouflage cream.

According to one embodiment of the present disclosure, the military camouflage cream may further contain at least one selected from the group consisting of triethylhexanoin, phenyl trimethicone, cyclopentasiloxane, cetyl PEG/PPG-10/1 dimethicone, trimethylsiloxysilicate, glyceryl caprylate, *Hippophae rhamnoides* oil, *Olea Europaea* (olive) fruit oil, *Persea gratissima* (avocado) oil, *Camellia japonica* seed oil, tocopheryl acetate, and combinations thereof.

According to one embodiment of the present disclosure, the content of triethylhexanoin may be 5 parts by weight to 15 parts by weight based on 100 parts by weight of the military camouflage cream, the content of phenyl trimethicone may be 5 parts by weight to 15 parts by weight based on 100 parts by weight of the military camouflage cream, the content of cyclopentasiloxane may be 6 parts by weight to 20 parts by weight based on 100 parts by weight of the military camouflage cream, the content of cetyl PEG/PPG-10/1 dimethicone may be 1 part by weight to 5 parts by weight based on 100 parts by weight of the military camouflage cream, the content of trimethylsiloxysilicate may be 1 part by weight to 5 parts by weight based on 100 parts by weight of the military camouflage cream, the content of glyceryl caprylate may be 1 part by weight to 5 parts by weight based on 100 parts by weight of the military camouflage cream, the content of *Hippophae rhamnoides* oil may be 0.5 parts by weight to 1.5 parts by weight based on 100 parts by weight of the military camouflage cream, the content of *Olea europaea* (olive) fruit oil may be 0.5 parts by weight to 1.5 parts by weight based on 100 parts by weight of the military camouflage cream, the content of *Persea gratissima* (avocado) oil may be 0.5 parts by weight to 1.5 parts by weight based on 100 parts by weight of the military camouflage cream, the content of *Camellia japonica* seed oil may be 0.5 parts by weight to 1.5 parts by weight based on 100 parts by weight of the military camouflage cream, and the content of tocopheryl acetate may be 0.1 parts by weight to 1.0 part by weight based on 100 parts by weight of the military camouflage cream.

According to one embodiment of the present disclosure, the military camouflage cream may further contain *Prunus domestica* seed oil.

According to one embodiment of the present disclosure, the military camouflage cream may further contain at least one selected from the group consisting of ethylhexyl methoxycinnamate, *Copernicia cerifera* (carnauba) wax, *Euphorbia cerifera* (candelilla) wax, titanium dioxide, glycerol, 1,2-hexanediol, ethylhexylglycerin, and combinations thereof.

According to one embodiment of the present disclosure, the content of ethylhexyl methoxycinnamate may be 1 part by weight to 3 parts by weight based on 100 parts by weight of the military camouflage cream, the content of *Copernicia cerifera* (carnauba) wax may be 1 part by weight to 5 parts by weight based on 100 parts by weight of the military camouflage cream, the content of *Euphorbia cerifera* (candelilla) wax may be 1 part by weight to 5 parts by weight based on 100 parts by weight of the military camouflage cream, the content of titanium dioxide may be 2 parts by weight to 6 parts by weight based on 100 parts by weight of the military camouflage cream, the content of glycerol may be 5 parts by weight to 15 parts by weight based on 100 parts by weight of the military camouflage cream, the content of 1,2-hexanediol may be 1 part by weight to 3 parts by weight based on 100 parts by weight of the military camouflage cream, and the content of ethylhexylglycerin may be 0.01 parts by weight to 0.10 parts by weight based on 100 parts by weight of the military camouflage cream.

According to one embodiment of the present disclosure, the military camouflage cream may further contain a pigment, wherein the pigment may be at least one selected from the group consisting of black iron oxide, red iron oxide, yellow iron oxide, and combinations thereof.

According to one embodiment of the present disclosure, the content of the pigment may be 0.5 parts by weight to 1.5 parts by weight based on 100 parts by weight of the military camouflage cream.

One embodiment of the present disclosure provides a military camouflage cream package including the military camouflage cream and having at least one color.

According to one embodiment of the present disclosure, the color may include one selected from the group consisting of khaki, brown, black, white, and combinations thereof.

The military camouflage cream according to one embodiment of the present disclosure may have improved spreadability, opacity, camouflage performance, and washing-out ability, and prevent skin problems.

The military camouflage cream package according to one embodiment of the present disclosure has at least one color and may exhibit improved camouflage performance by applying a desired pattern and color selected according to the surrounding environment.

### DETAILED DESCRIPTION

Throughout the present specification, it is to be understood that when any part is referred to as "containing" or "including" any component, it does not exclude other components, but may further contain or include other components, unless otherwise specified.

In the present specification, "A and/or B" means "A and B" or "A or B".

Hereinafter, the present disclosure will be described in more detail.

One embodiment of the present disclosure provides a military camouflage cream containing silica, talc, and kaolin.

The military camouflage cream according to one embodiment of the present disclosure may have improved spreadability, opacity, camouflage performance, and washing-out ability, and prevent skin problems.

According to one embodiment of the present disclosure, the military camouflage cream contains kaolin. As the military camouflage cream contains kaolin as described above, it may have a soft color, and have excellent spreadability, meaning that it may be applied to a wide area of the skin even when used in a small amount. In addition, it does not require drying time after application, and does not melt by the heat of the desert, and washing out thereof by water may be minimized.

According to one embodiment of the present disclosure, the kaolin may be derived from kaolinite. As the kaolin derived from the natural material kaolinite as described above is used, environmental pollution may be minimized and skin problems may be minimized by reducing skin toxicity.

According to one embodiment of the present disclosure, the content of kaolin may be 1.6 parts by weight to 2.8 parts by weight based on 100 parts by weight of the military camouflage cream. Specifically, the content of kaolin may be 1.7 parts by weight to 2.7 parts by weight, 1.8 parts by weight to 2.6 parts by weight, 1.9 parts by weight to 2.5 parts by weight, 2.0 parts by weight to 2.5 parts by weight, 1.8 parts by weight to 2.2 parts by weight, 1.9 parts by weight to 2.1 parts by weight, 2.3 parts by weight to 2.7 parts by weight, or 2.4 parts by weight to 2.6 parts by weight, based on 100 parts by weight of the military camouflage cream. As the content of kaolin is controlled within the above-described range, it may have a soft color, and have excellent spreadability, meaning that it may be applied to a wide area of the skin even when used in a small amount, does not require drying time after application, and does not melt by the heat of the desert, and washing out thereof by water may be minimized.

According to one embodiment of the present disclosure, the kaolinite may be one produced at Hadong Mine or Sancheong Mine (South Korea). As the kaolinite is selected from those described above, it is possible to control the components contained in the kaolinite and improve the oil absorption ability and opacity performance of the military camouflage cream.

According to one embodiment of the present disclosure, the kaolin may contain 45 wt% to 60 wt% of SiCt, 0.5 wt% to 1.5 wt% of FeO₃, 0.5 wt% to 1.5 wt% of K₂O-Na₂O, and 25 wt% to 40 wt% of Al₂O₃. As the contents of the components in the kaolin are controlled within the above ranges, it is possible to improve the oil absorption ability and opacity performance of the military camouflage cream and to select one suitable for use in appropriate places.

According to one embodiment of the present disclosure, the water content of the kaolin may be 1 wt% to 18 wt%. As the water content of the kaolin is controlled within the above range, it is possible to improve the dispersibility of the kaolin, improve spreadability, and minimize skin irritation.

According to one embodiment of the present disclosure, the kaolin may be calcined at a temperature of 1,100°C to 1,300°C. As used herein, the term "calcined" may mean heating to a specific temperature. As the kaolin is calcined at a temperature within the above range, it is possible to control the content of water in the kaolin and improve the dispersibility of the kaolin.

According to one embodiment of the present disclosure, the pH of the kaolin may be 5 to 7. As the pH of the kaolin is controlled within the above range, it is possible to prevent the skin from being damaged.

According to one embodiment of the present disclosure, the refractive index of the kaolin may be 1.2 to 1.8. As the refractive index of the kaolin is controlled within the above range, it is possible to improve the camouflage performance of the military camouflage cream.

According to an exemplary embodiment of the present disclosure, the kaolin may contain the components shown in Table 1 below and may be used for desert use, all-season use, snow use, subtropical use, or makeup use. As the kaolin containing the components shown in Table 1 below as described above is used, the opacity and color tone required in a specific place may be easily achieved.

**[Table 1]**

| | SiO₂ (wt%) | FeO₃ (wt%) | K-₂O-Na₂O (wt%) | Al₂O₃ (wt%) | Water (wt%) |
|---|---|---|---|---|---|
| For desert use | 49.00 | 0.80 | 0.80 | 36.00 | 2.00 |
| For all-season use | 48.00 | 0.80 | 0.80 | 38.00 | 15.00 |
| For snow use | 50.00 | 0.80 | 1.00 | 34.00 | 15.00 |
| For subtropical use | 48.00 | 1.00 | 0.60 | 37.00 | 15.00 |
| For makeup use | 58.00 | 0.60 | 0.80 | 28.00 | 15.00 |

According to one embodiment of the present disclosure, the particle size of the kaolin may be 1,000 mesh to 1,500 mesh. As the particle size of the kaolin is controlled within the above range, it is possible to improve the oil adsorption ability of the kaolin and improve the compatibility between the components of the military camouflage cream.

According to one embodiment of the present disclosure, the military camouflage cream contains silica. As the military camouflage cream contains silica as described above, it is possible to improve the spreadability and washing-out ability of the military camouflage cream.

According to one embodiment of the present disclosure, the content of silica may be 5.0 parts by weight to 9.0 parts by weight based on 100 parts by weight of the military camouflage cream. Specifically, the content of the silica may be 5.1 parts by weight to 8.9 parts by weight, 5.2 parts by weight to 8.8 parts by weight, 5.3 parts by weight to 8.7 parts by weight, 5.4 parts by weight to 8.6 parts by weight, 5.5 parts by weight to 8.5 parts by weight, 5.6 parts by weight to 8.4 parts by weight, 5.7 parts by weight to 8.3 parts by weight, 5.8 parts by weight to 8.2 parts by weight, 5.9 parts by weight to 8.1 parts by weight, or 6.0 parts by weight to 8.0 parts by weight, based on 100 parts by weight of the military camouflage cream. As the content of silica is controlled within the above range, it is possible to improve the spreadability and washing-out ability of the military camouflage cream.

According to one embodiment of the present disclosure, the military camouflage cream contains talc. As the military camouflage cream contains talc as described above, it is possible to improve the spreadability and washing-out ability of the military camouflage cream.

According to one embodiment of the present disclosure, the content of talc may be 3.5 parts by weight to 7.5 parts by weight based on 100 parts by weight of the military camouflage cream. Specifically, the content of talc may be 3.6 parts by weight to 7.4 parts by weight, 3.7 parts by weight to 7.3 parts by weight, 3.8 parts by weight to 7.2 parts by weight, 3.9 parts by weight to 7.1 parts by weight, or 4.0 parts by weight to 7.0 parts by weight, based on 100 parts by weight of the military camouflage cream. As the content of talc is controlled within the above range, it is possible to improve the spreadability and washing-out ability of the military camouflage cream.

According to one embodiment of the present disclosure, the military camouflage cream may further contain at least one selected from the group consisting of triethylhexanoin, phenyl trimethicone, cyclopentasiloxane, cetyl PEG/PPG-10/1 dimethicone, trimethylsiloxysilicate, cyclopentasiloxane, glyceryl caprylate, *Hippophae rhamnoides* oil, *Olea Europaea* (olive) fruit oil, *Persea gratissima* (avocado) oil, *Camellia japonica* seed oil, tocopheryl acetate, and combinations thereof. As the military camouflage cream contains the component described above, it is possible to minimize the toxicity of the military camouflage cream, prevent skin problems, and improve skin conditions.

According to one embodiment of the present disclosure, the military camouflage cream contains triethylhexanoin. As the military camouflage cream contains triethylhexanoin as described above, it is possible to provide conditioning to the skin, keep the skin moist by blocking evaporation of water from the skin, and keep the military camouflage cream unscented.

According to one embodiment of the present disclosure, the military camouflage cream contains phenyl trimethicone. As the military camouflage cream contains phenyl trimethicone as described above, it may form a barrier on the skin surface, thus reducing water loss from the skin.

According to one embodiment of the present disclosure, the military camouflage cream contains cyclopentasiloxane. As the military camouflage cream contains cyclopentasiloxane as described above, the cyclopentasiloxane improves spreadability, ensures even distribution of other components, delivers other components to the skin, and evaporates quickly from the skin surface, thus minimizing the heavy feeling.

According to one embodiment of the present disclosure, the military camouflage cream contains cetyl PEG/PPG-10/1 dimethicone. Specifically, cetyl PEG/PPG-10/1 dimethicone may be a copolymer of cetyl dimethicone and an alkoxylated derivative of dimethicone containing about 10 moles of polyethylene oxide and about 1 mole of propylene oxide. As the military camouflage cream contains cetyl PEG/PPG-10/1 dimethicone as described above, it is possible to improve the dispersibility in the product, improve the feeling of use, and maximize sustainability by improving adhesion.

According to one embodiment of the present disclosure, the military camouflage cream contains trimethylsiloxysilicate. As the military camouflage cream contains trimethylsiloxysilicate as described above, it is possible to prevent bubbles from being generated during the preparation of the military camouflage cream, minimize water evaporation from the skin, and prevent water from evaporating from the skin surface when applied.

According to one embodiment of the present disclosure, the military camouflage cream contains glyceryl caprylate. As the military camouflage cream contains glyceryl caprylate as described above, it is possible to add flexibility to the skin and improve the stability of the components.

According to one embodiment of the present disclosure, the military camouflage cream contains *Hippophae rhamnoides* oil. As the military camouflage cream contains *Hippophae rhamnoides* oil as described above, it may improve skin elasticity, soothe the skin, and have an improved skin moisturizing effect.

According to one embodiment of the present disclosure, the military camouflage cream contains *Olea europaea* (olive) fruit oil. As the military camouflage cream contains *Olea europaea* (olive) fruit oil as described above, it may make the skin soft and oily with a light and smooth texture and prevent evaporation of water from the skin.

According to one embodiment of the present disclosure, the military camouflage cream contains *Persea gratissima* (avocado) oil. As the military camouflage cream contains *Persea gratissima* (avocado) oil as described above, it may improve the moisturizing effect of adding oil to the skin and blocking water evaporation, and may be absorbed into the skin, thus exhibiting the effect of making the skin moist and shiny.

According to one embodiment of the present disclosure, the military camouflage cream contains *Camellia japonica* seed oil. As the military camouflage cream contains *Camellia japonica* seed oil as described above, it may eliminate reactive oxygen species and prevent cell senescence.

According to one embodiment of the present disclosure, the military camouflage cream contains tocopheryl acetate. As military camouflage cream contains tocopheryl acetate as described above, the cream may prevent and delay skin damage, reduce water loss from the skin, and may be prevented from being oxidized and deteriorated by combination with oxygen in the air.

According to one embodiment of the present disclosure, the content of triethylhexanoin may be 5 parts by weight to 15 parts by weight based on 100 parts by weight of the military camouflage cream, the content of phenyl trimethicone may be 5 parts by weight to 15 parts by weight based on 100 parts by weight of the military camouflage cream, the content of cyclopentasiloxane may be 6 parts by weight to 20 parts by weight based on 100 parts by weight of the military camouflage cream, the content of cetyl PEG/PPG-10/1 dimethicone may be 1 part by weight to 5 parts by weight based on 100 parts by weight of the military camouflage cream, the content of trimethylsiloxysilicate may be 1 part by weight to 5 parts by weight based on 100 parts by weight of the military camouflage cream, the content of glyceryl caprylate may be 1 part by weight to 5 parts by weight based on 100 parts by weight of the military camouflage cream, the content of *Hippophae rhamnoides* oil may be 0.5 parts by weight to 1.5 parts by weight based on 100 parts by weight of the military camouflage cream, the content of *Olea europaea* (olive) fruit oil may be 0.5 parts by weight to 1.5 parts by weight based on 100 parts by weight of the military camouflage cream, the content of *Persea gratissima* (avocado) oil may be 0.5 parts by weight to 1.5 parts by weight based on 100 parts by weight of the military camouflage cream, the content of *Camellia japonica* seed oil may be 0.5 parts by weight to 1.5 parts by weight based on 100 parts by weight of the military camouflage cream, and the content of tocopheryl acetate may be 0.1 parts by weight to 1.0 part by weight based on 100 parts by weight of the military camouflage cream.

According to one embodiment of the present disclosure, the content of triethylhexanoin may be 5 parts by weight to 15 parts by weight based on 100 parts by weight of the military camouflage cream. Specifically, the content of triethylhexanoin may be 6 parts by weight to 14 parts by weight, 7 parts by weight to 13 parts by weight, 8 parts by weight to 12 parts by weight, or 9 parts by weight to 11 parts by weight, based on 100 parts by weight of the military camouflage cream. As the content of triethylhexanoin is controlled within the above range, it is possible to provide conditioning to the skin, maintain moist skin by blocking water evaporation from the skin, and give a good scent to the military camouflage cream.

According to one embodiment of the present disclosure, the content of phenyl trimethicone may be 5 parts by weight to 15 parts by weight based on 100 parts by weight of the military camouflage cream. Specifically, the content of phenyl trimethicone may be 6 parts by weight to 14 parts by weight, 7 parts by weight to 13 parts by weight, 8 parts by weight to 12 parts by weight, or 9 parts by weight to 11 parts by weight, based on 100 parts by weight of the military camouflage cream. As the content of phenyl trimethicone is controlled within the above range, it is possible to form a barrier on the skin surface, thus reducing water loss from the skin.

According to one embodiment of the present disclosure, the content of cyclopentasiloxane may be 6 parts by weight to 25 parts by weight based on 100 parts by weight of the military camouflage cream. Specifically, the content of cyclopentasiloxane may be 6 parts by weight to 14 parts by weight, 7 parts by weight to 13 parts by weight, 8 parts by weight to 12 parts by weight, or 9 parts by weight to 11 parts by weight, based on 100 parts by weight of the military camouflage cream. As the content of cyclopentasiloxane is controlled within the above range, the cyclopentasiloxane improves spreadability, ensures even distribution of other components, delivers other components to the skin, and evaporates quickly from the skin surface, thus minimizing the heavy feeling.

According to one embodiment of the present disclosure, the content of cetyl PEG/PPG-10/1 dimethicone may be 1 part by weight to 5 parts by weight based on 100 parts by weight of the military camouflage cream. Specifically, the content of cetyl PEG/PPG-10/1 dimethicone may be 2 parts by weight to 4 parts by weight based on 100 parts by weight of the military camouflage cream. As the content of cetyl PEG/PPG-10/1 dimethicone is controlled within the above range, it is possible to improve the dispersibility in the product, improve the feeling of use, and maximize sustainability by improving adhesion.

According to one embodiment of the present disclosure, the content of trimethylsiloxysilicate may be 1 part by weight to 5 parts by weight based on 100 parts by weight of the military camouflage cream. Specifically, the content of trimethylsiloxysilicate may be 2 parts by weight to 4 parts by weight based on 100 parts by weight of the military camouflage cream. As the content of the trimethylsiloxysilicate is controlled within the above range, it is possible to prevent bubbles from being generated during the preparation of the military camouflage cream, minimize water evaporation, and prevent water from evaporating from the skin surface when applied.

According to one embodiment of the present disclosure, the content of glyceryl caprylate may be 1 part by weight to 5 parts by weight based on 100 parts by weight of the military camouflage cream. Specifically, the content of the glyceryl caprylate may be 2 parts by weight to 4 parts by weight based on 100 parts by weight of the military camouflage cream. As the content of glyceryl caprylate is controlled within the above-mentioned range, it is possible to add flexibility to the skin and improve the stability of the components.

According to one embodiment of the present disclosure, the content of *Hippophae rhamnoides* oil may be 0.5 parts by weight to 1.5 parts by weight based on 100 parts by weight of the military camouflage cream. Specifically, the content of *Hippophae rhamnoides* oil may be 0.6 parts by weight to 1.4 parts by weight, 0.7 parts by weight to 1.3 parts by weight, 0.8 parts by weight to 1.2 parts by weight, or 0.9 parts by weight to 1.1 parts by weight, based on 100 parts by weight of the military camouflage cream. As the content of *Hippophae rhamnoides* oil is controlled within the above range, it is possible to improve skin elasticity, soothe the skin, and improve the skin moisturizing effect.

According to one embodiment of the present disclosure, the content of *Olea europaea* (olive) fruit oil may be 0.5 parts by weight to 1.5 parts by weight based on 100 parts by weight of the military camouflage cream. Specifically, the content of *Olea europaea* (olive) fruit oil may be 0.6 parts by weight to 1.4 parts by weight, 0.7 parts by weight to 1.3 parts by weight, 0.8 parts by weight to 1.2 parts by weight, or 0.9 parts by weight to 1.1 parts by weight, based on 100 parts by weight of the military camouflage cream. As the content of *Olea europaea* (olive) fruit oil is controlled within the above range, it is possible to make the skin soft and oily with a light and smooth texture and prevent evaporation of water from the skin.

According to one embodiment of the present disclosure, the content of *Persea gratissima* (avocado) oil may be 0.5 parts by weight to 1.5 parts by weight based on 100 parts by weight of the military camouflage cream. Specifically, the content of *Persea gratissima* (avocado) oil may be 0.6 parts by weight to 1.4 parts by weight, 0.7 parts by weight to 1.3 parts by weight, 0.8 parts by weight to 1.2 parts by weight, or 0.9 parts by weight to 1.1 parts by weight, based on 100 parts by weight of the military camouflage cream. As the content of *Persea gratissima* (avocado) oil is controlled within the above range, the *Persea gratissima* (avocado) oil may improve the moisturizing effect of adding oil to the skin and blocking water evaporation from the skin, and may be absorbed into the skin, thus exhibiting the effect of making the skin moist and shiny.

According to one embodiment of the present disclosure, the content of *Camellia japonica* seed oil may be 0.5 parts by weight to 1.5 parts by weight based on 100 parts by weight of the military camouflage cream. Specifically, the content of *Camellia japonica* seed oil may be 0.6 parts by weight to 1.4 parts by weight, 0.7 parts by weight to 1.3 parts by weight, 0.8 parts by weight to 1.2 parts by weight, or 0.9 parts by weight to 1.1 parts by weight, based on 100 parts by weight of the military camouflage cream. As the content of *Camellia japonica* seed oil is controlled within the above range, it is possible to eliminate reactive oxygen species and prevent cell senescence.

According to one embodiment of the present disclosure, the content of tocopheryl acetate may be 0.1 part by weight to 1.0 part by weight based on 100 parts by weight of the military camouflage cream. Specifically, the content of tocopheryl acetate may be 0.2 parts by weight to 0.9 parts by weight, 0.3 parts by weight to 0.8 parts by weight, 0.4 parts by weight to 0.7 parts by weight, or 0.5 parts by weight to 0.6 parts by weight, based on 100 parts by weight of the military camouflage cream. As the content of tocopheryl acetate is controlled within the above range, it is possible to prevent and delay skin damage, reduce water loss from the skin, and prevent the cream from being oxidized and deteriorated by combination with oxygen in the air.

According to one embodiment of the present disclosure, the military camouflage cream may further contain *Prunus domestica* seed oil. As the military camouflage cream further contains *Prunus domestica* seed oil as described above, it may improve skin flexibility.

According to one embodiment of the present disclosure, the military camouflage cream may further contain at least one selected from the group consisting of ethylhexyl methoxycinnamate, *Copernicia cerifera* (carnauba) wax, *Euphorbia cerifera* (candelilla) wax, titanium dioxide, glycerol, 1,2-hexanediol, ethylhexylglycerin, and combinations thereof. As the military camouflage cream further contains the above component as described above, it is possible to improve spreadability, opacity, camouflage performance, and washing-out ability, and prevent skin problems.

According to one embodiment of the present disclosure, the military camouflage cream may contain ethylhexyl methoxycinnamate. As the military camouflage cream contains ethylhexyl methoxycinnamate as described above, ethylhexyl methoxycinnamate, it is possible to prevent the skin from being irradiated with ultraviolet rays.

According to one embodiment of the present disclosure, the military camouflage cream may contain *Copernicia cerifera* (carnauba) wax. As the military camouflage cream contains *Copernicia cerifera* (carnauba) wax as described above, it may revitalize the skin, improve skin flexibility, and prevent water in the skin from evaporating.

According to one embodiment of the present disclosure, the military camouflage cream may contain *Euphorbia cerifera* (candelilla) wax. As the military camouflage cream contains *Euphorbia cerifera* (candelilla) wax as described above, the cream may form a skin protective film that gently covers and protects rough skin and lips, add gloss to the skin, provide flexibility to the skin by improving skin texture, and prevent evaporation of water from the skin, and the viscosity thereof may be controlled.

According to one embodiment of the present disclosure, the military camouflage cream may contain titanium dioxide. As the military camouflage cream contains titanium dioxide as described above, the cream may block ultraviolet rays and visible rays and prevent skin aging.

According to one embodiment of the present disclosure, the military camouflage cream may contain glycerol. As the military camouflage cream contains glycerol as described above, the cream may exhibit the effect of retaining water in the skin.

According to one embodiment of the present disclosure, the military camouflage cream may contain 1,2-hexanediol. As the military camouflage cream contains 1,2-hexanediol as described above, the cream may have improved moisturizing ability and antibacterial activity.

According to one embodiment of the present disclosure, the military camouflage cream may contain ethylhexylglycerin. As the military camouflage cream contains ethylhexylglycerin as described above, the ethylhexylglycerin may revitalize the skin, act as a solvent to dissolve other components, increase antibacterial activity, and inhibit the growth of anaerobic bacteria.

According to one embodiment of the present disclosure, the content of ethylhexyl methoxycinnamate may be 1 part by weight to 3 parts by weight based on 100 parts by weight of the military camouflage cream, the content of *Copernicia cerifera* (carnauba) wax may be 1 part by weight to 5 parts by weight based on 100 parts by weight of the military camouflage cream, the content of *Euphorbia cerifera* (candelilla) wax may be 1 part by weight to 5 parts by weight based on 100 parts by weight of the military camouflage cream, the content of titanium dioxide may be 2 parts by weight to 6 parts by weight based on 100 parts by weight of the military camouflage cream, the content of glycerol may be 5 parts by weight to 15 parts by weight based on 100 parts by weight of the military camouflage cream, the content of 1,2-hexanediol may be 1 part by weight to 3 parts by weight based on 100 parts by weight of the military camouflage cream, and the content of ethylhexylglycerin may be 0.01 parts by weight to 0.10 parts by weight based on 100 parts by weight of the military camouflage cream.

According to one embodiment of the present disclosure, the content of ethylhexyl methoxycinnamate may be 1 part by weight to 3 parts by weight based on 100 parts by weight of the military camouflage cream. Specifically, the content of ethylhexyl methoxycinnamate may be 1.2 parts by weight to 2.8 parts by weight, 1.4 parts by weight to 2.6 parts by weight, 1.6 parts by weight to 2.4 parts by weight or less, or 1.8 parts by weight to 2.2 parts by weight, based on 100 parts by weight of the military camouflage cream. As the content of ethylhexyl methoxycinnamate is controlled within the above range, it is possible to prevent the skin from being irradiated with ultraviolet rays.

According to one embodiment of the present disclosure, the content of *Copernicia cerifera* (carnauba) wax may be 1 part by weight to 5 parts by weight based on 100 parts by weight of the military camouflage cream. Specifically, the content of *Copernicia cerifera* (carnauba) wax may be 1.5 parts by weight to 4.5 parts by weight, 2.0 parts by weight to 4.0 parts by weight, or 2.5 parts by weight to 3.5 parts by weight, based on 100 parts by weight of the military camouflage cream. As the content of *Copernicia cerifera* (carnauba) wax is controlled within the above range, it is possible to revitalize the skin, improve skin flexibility, and prevent water in the skin from evaporating.

According to one embodiment of the present disclosure, the content of *Euphorbia cerifera* (candelilla) wax may be 1 part by weight to 5 parts by weight based on 100 parts by weight of the military camouflage cream. Specifically, the content of *Euphorbia cerifera* (candelilla) wax may be 1.5 parts by weight to 4.5 parts by weight, 2.0 parts by weight to 4.0 parts by weight, or 2.5 parts by weight to 3.5 parts by weight, based on 100 parts by weight of the military camouflage cream. As the content of *Euphorbia cerifera* (candelilla) wax is controlled within the above range, it is possible to form a skin protective film that gently covers and protects rough skin and lips, add gloss to the skin, provide flexibility to the skin by improving skin texture, prevent evaporation of water from the skin, and control the viscosity of the cream.

According to one embodiment of the present disclosure, the content of titanium dioxide may be 2 parts by weight to 6 parts by weight based on 100 parts by weight of the military camouflage cream. Specifically, the content of titanium dioxide may be 2.5 parts by weight to 5.5 parts by weight, 3.0 parts by weight to 5.0 parts by weight, or 3.5 parts by weight to 4.5 parts by weight, based on 100 parts by weight of the military camouflage cream. As the content of titanium dioxide is controlled within the above-mentioned range, it is possible to block ultraviolet rays and visible rays and prevent skin aging.

According to one embodiment of the present disclosure, the content of glycerol may be 5 parts by weight to 15 parts by weight based on 100 parts by weight of the military camouflage cream. Specifically, the content of glycerol may be 6 parts by weight to 14 parts by weight, 7 parts by weight to 13 parts by weight, 8 parts by weight to 12 parts by weight, or 9 parts by weight to 11 parts by weight, based on 100 parts by weight of the military camouflage cream. As the content of glycerol is controlled within the above range, it is possible to achieve the effect of retaining water in the skin.

According to one embodiment of the present disclosure, the content of 1,2-hexanediol may be 1 part by weight to 3 parts by weight based on 100 parts by weight of the military camouflage cream. Specifically, the content of 1,2-hexanediol may be 1.2 parts by weight to 2.8 parts by weight, 1.4 parts by weight to 2.6 parts by weight, 1.6 parts by weight to 2.4 parts by weight, or 1.8 parts by weight to 2.2 parts by weight, based on 100 parts by weight of the military camouflage cream. As the content of 1,2-hexanediol is controlled within the above range, it is possible to improve the moisturizing ability and antibacterial activity of the cream.

According to one embodiment of the present disclosure, the content of ethylhexylglycerin may be 0.01 parts by weight to 0.10 parts by weight based on 100 parts by weight of the military camouflage cream. Specifically, the content of ethylhexylglycerin may be 0.02 parts by weight to 0.09 parts by weight, 0.03 parts by weight to 0.08 parts by weight, 0.04 parts by weight to 0.07 parts by weight, or 0.05 parts by weight to 0.06 parts by weight, based on 100 parts by weight of the military camouflage cream. As the content of ethylhexylglycerin is controlled within the above range, the ethylhexylglycerin may revitalize the skin, act as a solvent to dissolve other components, increase antibacterial activity, and inhibit the growth of anaerobic bacteria.

According to one embodiment of the present disclosure, the military camouflage cream may further contain a pigment, wherein the pigment may be at least one selected from the group consisting of black iron oxide, red iron oxide, yellow iron oxide, and combinations thereof. As the military camouflage cream contains the pigment as described above and a specific pigment is selected, it is possible to minimize skin irritation, easily achieve a desired color, and improve camouflage performance.

According to one embodiment of the present disclosure, the content of the pigment may be 0.5 parts by weight to 1.5 parts by weight based on 100 parts by weight of the military camouflage cream. Specifically, the content of the pigment may be 0.6 parts by weight to 1.4 parts by weight, 0.7 parts by weight to 1.3 parts by weight, 0.8 parts by weight to 1.2 parts by weight, or 0.9 parts by weight to 1.1 parts by weight, based on 100 parts by weight of the military camouflage cream. As the content of the pigment is controlled within the above range, it is possible to achieve a desired color of the military camouflage cream and at the same time, minimize skin irritation.

According to one embodiment of the present disclosure, the military camouflage cream may further contain purified water as the remainder. As the military camouflage cream further contains purified water as the remainder, it is possible to control the viscosity of the military camouflage cream.

According to one embodiment of the present disclosure, the kaolin may have a whiteness (L*) of 86 or more. Specifically, the kaolin may have a whiteness (L*) of 90, 92, 94, or 95. When colors are combined, the kaolin may have a whiteness (L*) of 100 or less, 99 or less, 98 or less, or 97 or less. The whiteness (L*) may be measured using the Lab* color space, and the whiteness (L*) may be measured using the spectrophotometer MINOLTA CM-3700d (light source D/10).

According to one embodiment of the present disclosure, the kaolin allows the externally exposed surface of the skin to radiate heat energy in the form of electromagnetic waves, or allows the excessive temperature rise of the skin surface layer in response to a heat-emitting weapon to be fundamentally suppressed regardless of the color or contamination thereof. Furthermore, when the kaolin and titanium oxide are added, they exhibit the effect of blocking conductive heat from infrared/ultraviolet rays as a heat emitter and show a heat reduction of 1°C or less.

One embodiment of the present disclosure provides a military camouflage cream package including the military camouflage cream and having at least one color.

The military camouflage cream package according to one embodiment of the present disclosure has at least one color and may exhibit improved camouflage performance by applying a desired pattern and color selected according to the surrounding environment.

According to one embodiment of the present disclosure, the military camouflage cream package may have a color selected from the group consisting of khaki, brown, black, white, and combinations thereof.

Hereinafter, the present disclosure will be described in detail by way of examples. However, the embodiments according to the present disclosure may be modified in various different forms, and the scope of the present disclosure is not to be construed as being limited to the examples described below. The examples of the present specification are provided to more completely explain the present disclosure to those of ordinary skill in the art.

### <Examples and Comparative Examples>

Military camouflage creams were prepared using the composition ratios shown in Table 2 below. Specifically, an oil phase was prepared by mixing triethylhexanoin, phenyl trimethicone, cyclopentasiloxane, cetyl PEG/PPG-10/1 dimethicone, trimethylsiloxysilicate, glyceryl caprylate, *Hippophae rhamnoides* oil, *Olea europaea* (olive) fruit oil, *Persea gratissima* (avocado) oil, *Camellia japonica* seed oil, and tocopheryl acetate, and dissolving the mixture at 70°C to 80°C for 3 minutes.

Then, a water phase was prepared by mixing ethylhexyl methoxycinnamate, *Copernicia cerifera* (carnauba) wax, *Euphorbia cerifera* (candelilla) wax, purified water, titanium dioxide, silica, talc, kaolin, black iron oxide, glycerol, 1,2-hexanediol, and ethylhexylglycerin, and dissolving the mixture at 70°C to 80°C for 3 minutes.

Thereafter, the oil phase and the water phase were mixed together, and the mixture was emulsified with an azimixer, stirred with a homomixer, cooled rapidly to 45°C within 10 minutes, and then debubbled, thus preparing military camouflage cream.

**[Table 2]**

| Components | Comparative Example 1 | Comparative Example 2 | Example 1 | Example 2 |
|---|---|---|---|---|
| Triethylhexanoin | 10.00 | 10.00 | 10.00 | 10.00 |
| Phenyl trimethicone | 10.00 | 10.00 | 10.00 | 10.00 |
| Cyclopentasiloxane | 13.00 | 13.00 | 13.00 | 13.00 |
| Cetyl PEG/PPG-10/1 dimethicone | 3.00 | 3.00 | 3.00 | 3.00 |
| Trimethylsiloxysilicate | 3.00 | 3.00 | 3.00 | 3.00 |
| Glyceryl caprylate | 3.00 | 3.00 | 3.00 | 3.00 |
| Hippophae rhamnoides oil | 1.00 | 1.00 | 1.00 | 1.00 |
| *Olea europaea*(olive) fruit oil | 1.00 | 1.00 | 1.00 | 1.00 |
| *Persea Gratissima(avocado)* oil | 1.00 | 1.00 | 1.00 | 1.00 |
| *Camellia japonica* seed oil | 1.00 | 1.00 | 1.00 | 1.00 |
| Tocopheryl acetate | 0.50 | 0.50 | 0.50 | 0.50 |
| Ethylhexyl methoxycinnamate | 2.00 | 2.00 | 2.00 | 2.00 |
| *Copernicia cerifera* (carnauba) wax | 3.00 | 3.00 | 3.00 | 3.00 |
| *Euphorbia cerifera* (candelilla) wax | 3.00 | 3.00 | 3.00 | 3.00 |
| Purified water | Remainder | Remainder | Remainder | Remainder |
| Titanium dioxide | 4.00 | 4.00 | 4.00 | 4.00 |
| Silica | 2.00 | 4.00 | 6.00 | 8.00 |
| Talc | 1.00 | 3.00 | 4.00 | 7.00 |
| Kaolin | 1.00 | 1.50 | 2.00 | 2.50 |
| Pigment (black iron oxide) | 1.00 | 1.00 | 1.00 | 1.00 |
| Glycerol | 10.00 | 10.00 | 10.00 | 10.00 |
| 1,2-Hexanediol | 2.00 | 2.00 | 2.00 | 2.00 |
| Ethylhexylglycerin | 0.05 | 0.05 | 0.05 | 0.05 |

### <Experimental Example 1: Evaluation of Opacity and Spreadability>

In order to examine the degrees of opacity and spreadability of Examples 1 and 2 and Comparative Examples 1 and 2, the feeling of use (opacity upon initial application and spreadability upon finishing) was evaluated on 9 men in their 0s to 30s by a panel test, and the results are shown in Table 3 below.

"Opacity" refers to the degree of coloring when started to apply. A higher opacity score indicates better coloring, and a lower opacity score indicates a lower degree of coloring (unsuitable - almost not colored, good - colored to some extent, excellent - well colored with a dark color).

"Spreadability" refers to the degree to which cosmetics are spread smoothly when applied to the skin. A higher score indicates smoother spreadability, and a lower score indicates harder spreadability (unsuitable - hard, good - smooth spread, excellent - very smoothly spread).

**[Table 3]**

| | Opacity | Spreadability |
|---|---|---|
| Example 1 | Good | Excellent |
| Example 2 | Excellent | Unsuitable |
| Comparative Example 1 | Unsuitable | Unsuitable |
| Comparative Example 2 | Good | Good |

### <Experimental Example 2: Evaluation of Washing-out Ability>

The washing-out abilities of Examples 1 and 2 and Comparative Examples 1 and 2 were evaluated. Evaluation of the washing-out ability was performed according to the following method.

Each of Examples 1 and 2 and Comparative Examples 1 and 2 was applied to the test area (inside the lower arm) in an amount of 2 µL/cm², and then the applied area was washed with water while rolling 30 times. Then, the applied area after washing was compared with the applied area before washing. The washing-out ability was evaluated according to the following criteria: unsuitable = a significant amount remained, good = a very small amount remained, excellent = almost no residue remained. The results of the evaluation are summarized in Table 4 below.

It can be seen that the compositions of Examples 1 and 2 showed an excellent ability to be washed out by a simple washing method such as water washing while rolling 30 times, whereas the compositions of Comparative Examples 1 and 2 showed the ability to be washed out while leaving residue.

**[Table 4]**

| | Washing-out ability |
|---|---|
| Example 1 | Excellent |
| Example 2 | Excellent |
| Comparative Example 1 | Good |
| Comparative Example 2 | Good |

### <Experimental Example 3: Measurement of Reduction Rate of Radiant Heat>

The radiant heat reduction effect of each of Examples 1 and 2 and Comparative Examples 1 and 2 was measured on 9 men in their 20s and 30s.

Before exposure to infrared rays, the subjects waited for 30 minutes under constant temperature and humidity conditions maintained at room temperature (about 25°C) and a relative humidity (RH) of 40% to 45%. Then, the subject's test area (upper arm) was exposed to direct sunlight for 20 minutes, and each of Examples 1 and 2 and Comparative Examples 1 and 2 was applied to the selected test area (2.5 cm x 2.5 cm) of the forearm in an amount of 10 µL by using a micropipette.

The results of measuring the temperature change of the skin with a Thermo-graphic Camera (FLIR, SWEDEN) before application of each of Examples 1 and 2 and Comparative Examples 1 and 2, immediately after application, 5 minutes after application, and 10 minutes after application were calculated according to Equation 1 below, and the results of the calculation are shown in Table 5 below. Radiant heat reduction rate (%) = {1- temperature of sample-applied area / temperature of non-sample-applied area} X 100

**[Table 5]**

| | Immediately after application (C) | After 5 minutes (°C) | After 10 minutes (°C) | Average (°C) | Result (%) |
|---|---|---|---|---|---|
| Untreated area | 34.9 | 34.8 | 35 | 34.9 | |
| Example 1 | 30.3 | 30.1 | 31.7 | 30.7 | 12.03 |
| Example 2 | 29.9 | 29.7 | 31.8 | 30.5 | 12.70 |
| Comparative Example 1 | 32.9 | 33.1 | 34.3 | 33.4 | 4.20 |
| Comparative Example 2 | 31.3 | 31.9 | 33 | 32.1 | 8.12 |

As shown in Table 5 above, it was confirmed that the radiant heat reduction effect was up to about 8% higher when the camouflage cream of each of Examples 1 and 2 was applied to the skin than when the camouflage cream of each of Comparative Examples 1 and 2 was applied to the skin.

### <Experimental Example 4: Evaluation of UV-Shielding Ability>

Each of Examples 1 and 2 and Comparative Examples 1 and 2 was diluted to concentrations of 1 wt%, 5 wt%, and 10 wt%, and irradiated with UV light at a wavelength of 320 nm to 400 nm using a black light UV lamp (ROBUST UV 12LED).

Water was used as a control. The experimental results are summarized in Table 6 below.

It could be seen that the samples of Example 1 at concentrations of 5 wt% and 10 wt% did not transmit UV light, and the sample of Example 1 at 1 wt% slightly transmitted UV light. As a result of performing the same experiment on Example 2, it could be seen that the samples of Example 2 hardly transmitted UV light even at a concentration of 1 wt%.

On the other hand, it could be seen that the samples of Comparative Examples 1 and 2 transmitted UV light at a concentration of 1 wt%, and the samples of Comparative Example 1 slightly transmitted UV light even at a concentration of 5 wt%.

**[Table 6]**

| | 1 wt% concentration | 5 wt% concentration | 10 wt% concentration |
|---|---|---|---|
| Example 1 | Slightly transmitted UV light | Did not transmit UV light | Did not transmit UV light |
| Example 2 | Did not transmit UV light | Did not transmit UV light | Did not transmit UV light |
| Comparative Example 1 | Transmitted UV light | Slightly transmitted UV light | Did not transmit UV light |
| Comparative Example 2 | Transmitted UV light | Did not transmit UV light | Did not transmit UV light |

The results of Experimental Examples 1 to 4 are summarized in Table 7 below.

| | Opacity | Spreadability | Washing-out ability | Radiant heat reduction rate | UV-shielding ability |
|---|---|---|---|---|---|
| Example 1 | Good | Excellent | Excellent | Excellent | Excellent |
| Example 2 | Excellent | Good | Excellent | Excellent | Excellent |
| Comparative Example 1 | Unsuitable | Unsuitable | Good | Unsuitable | Unsuitable |
| Comparative Example 2 | Good | Good | Good | Unsuitable | Unsuitable |

Referring to Table 7 above, it was confirmed that Examples 1 and 2 were good or excellent in terms of all of opacity, spreadability, washing-out ability, radiant heat reduction rate, and UV-shielding ability. On the other hand, it was confirmed that Comparative Example 1 was unsuitable in terms of in opacity, spreadability, radiant heat reduction rate, and UV-shielding ability, and Comparative Example 2 was unsuitable in terms of radiant heat reduction rate and UV-shielding ability.

Therefore, the military camouflage cream according to one embodiment of the present disclosure and the military camouflage cream package including the same contain silica, talc, and kaolin, the contents of which may be controlled to improve all of opacity, spreadability, washing-out ability, radiant heat reduction rate, and UV-shielding ability.

## Claims

1. A military camouflage cream containing silica, talc, and kaolin.

2. The military camouflage cream according to claim 1, wherein a content of the kaolin is 1.6 parts by weight to 2.8 parts by weight based on 100 parts by weight of the military camouflage cream.

3. The military camouflage cream according to claim 2, wherein a content of the silica is 5.0 parts by weight to 9.0 parts by weight based on 100 parts by weight of the military camouflage cream, and a content of the talc is 3.5 parts by weight to 7.5 parts by weight based on 100 parts by weight of the military camouflage cream.

4. The military camouflage cream according to claim 1, further containing at least one selected from the group consisting of triethylhexanoin, phenyl trimethicone, cyclopentasiloxane, cetyl PEG/PPG-10/1 dimethicone, trimethylsiloxysilicate, glyceryl caprylate, *Hippophae rhamnoides* oil, *Olea Europaea* (olive) fruit oil, *Persea gratissima* (avocado) oil, *Camellia japonica* seed oil, tocopheryl acetate, and combinations thereof.

5. The military camouflage cream according to claim 4, wherein
a content of the triethylhexanoin is 5 parts by weight to 15 parts by weight based on 100 parts by weight of the military camouflage cream,
a content of the phenyl trimethicone is 5 parts by weight to 15 parts by weight based on 100 parts by weight of the military camouflage cream,
a content of the cyclopentasiloxane is 6 parts by weight to 20 parts by weight based on 100 parts by weight of the military camouflage cream,
a content of the cetyl PEG/PPG-10/1 dimethicone is 1 part by weight to 5 parts by weight based on 100 parts by weight of the military camouflage cream,
a content of the trimethylsiloxysilicate is 1 part by weight to 5 parts by weight based on 100 parts by weight of the military camouflage cream,
a content of the glyceryl caprylate is 1 part by weight to 5 parts by weight based on 100 parts by weight of the military camouflage cream,
a content of the *Hippophae rhamnoides* oil is 0.5 parts by weight to 1.5 parts by weight based on 100 parts by weight of the military camouflage cream,
a content of the *Olea europaea* (olive) fruit oil is 0.5 parts by weight to 1.5 parts by weight based on 100 parts by weight of the military camouflage cream,
a content of the *Persea gratissima* (avocado) oil is 0.5 parts by weight to 1.5 parts by weight based on 100 parts by weight of the military camouflage cream,
a content of the *Camellia japonica* seed oil is 0.5 parts by weight to 1.5 parts by weight based on 100 parts by weight of the military camouflage cream, and
a content of the tocopheryl acetate is 0.1 parts by weight to 1.0 part by weight based on 100 parts by weight of the military camouflage cream.

6. The military camouflage cream according to claim 4, further containing *Prunus domestica* seed oil.

7. The military camouflage cream according to claim 4, further containing at least one selected from the group consisting of ethylhexyl methoxycinnamate, *Copernicia cerifera* (carnauba) wax, *Euphorbia cerifera* (candelilla) wax, titanium dioxide, glycerol, 1,2-hexanediol, ethylhexylglycerin, and combinations thereof.

8. The military camouflage cream according to claim 7, wherein
a content of the ethylhexyl methoxycinnamate is 1 part by weight to 3 parts by weight based on 100 parts by weight of the military camouflage cream,
a content of the *Copernicia cerifera* (carnauba) wax is 1 part by weight to 5 parts by weight based on 100 parts by weight of the military camouflage cream,
a content of the *Euphorbia cerifera* (candelilla) wax is 1 part by weight to 5 parts by weight based on 100 parts by weight of the military camouflage cream,
a content of the titanium dioxide is 2 parts by weight to 6 parts by weight based on 100 parts by weight of the military camouflage cream,
a content of the glycerol is 5 parts by weight to 15 parts by weight based on 100 parts by weight of the military camouflage cream,
a content of the 1,2-hexanediol is 1 part by weight to 3 parts by weight based on 100 parts by weight of the military camouflage cream, and
a content of the ethylhexylglycerin is 0.01 parts by weight to 0.10 parts by weight based on 100 parts by weight of the military camouflage cream.

9. The military camouflage cream according to claim 1, further containing a pigment, wherein the pigment comprises at least one selected from the group consisting of black iron oxide, red iron oxide, yellow iron oxide, and combinations thereof.

10. The military camouflage cream according to claim 9, wherein a content of the pigment is 0.5 parts by weight to 1.5 parts by weight based on 100 parts by weight of the military camouflage cream.

11. A military camouflage cream package comprising the military camouflage cream according to claim 1 and having at least one color.

12. The military camouflage cream package according to claim 11, wherein the color comprises one selected from the group consisting of khaki, brown, black, white, and combinations thereof.
